## Europäisches Patentamt

## European Patent Office
## Office européen des brevets

(19)

(11) Veröffentlichungsnummer: **0 025 982**
**A2**

(12) **EUROPÄISCHE PATENTANMELDUNG**

(21) Anmeldenummer: **80105601.1**

(22) Anmeldetag: **18.09.80**

(51) Int. Cl.³: **A 61 B 6/02**

(30) Priorität: **21.09.79 DE 2938289**

(43) Veröffentlichungstag der Anmeldung:
**01.04.81 Patentblatt 81/13**

(84) Benannte Vertragsstaaten:
**AT BE CH DE FR GB IT LI NL SE**

(71) Anmelder: **SIEMENS AKTIENGESELLSCHAFT Berlin und München**
**Postfach 22 02 61**
**D-8000 München 22(DE)**

(72) Erfinder: **Tschunt, Edgar**
**Im Winkel 9**
**D-8521 Rathsberg(DE)**

(54) **Schichtgerät zur Herstellung von Transversalschichtbildern eines Aufnahmeobjektes.**

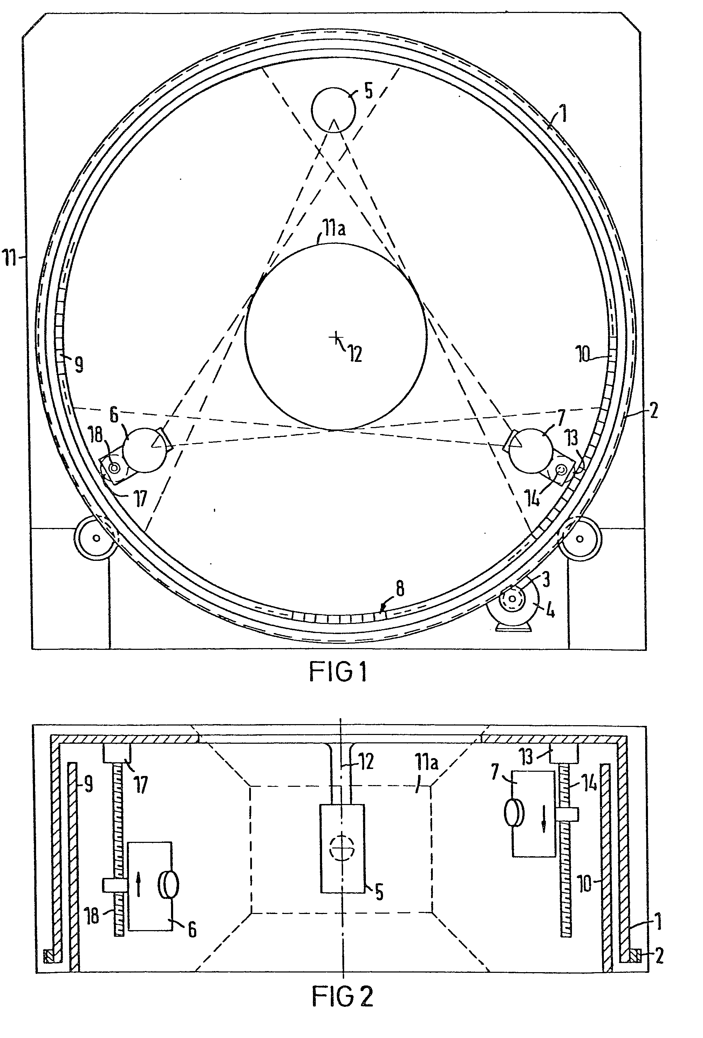

(57) Die Erfindung bezieht sich auf ein Schichtgerät zur Herstellung von Transversalschichtbildern eines Aufnahmeobjektes mit mehreren, mit ihren Fokussen um gleich große Winkel gegeneinander versetzt angeordneten Strahlenquellen (5, 6, 7), mit einer Röntgenstrahlenmeßanordnung, welche die Strahlungsintensität hinter dem Objekt ermittelt, mit einer Antriebsvorrichtung (3, 4), die einen Drehrahmen (1) zur Erzeugung von Drehbewegungen der Strahlenquellen (5, 6, 7) antreibt, sowie mit einem Meßwertumformer für die Transformation der von der Strahlenmeßanordnung (8) gelieferten Signale in ein Schichtbild. Die Strahlenquellen (5, 6, 7) sind in Richtung der Drehachse (12) mit ihren Fokussen in im Abstand zueinander liegenden Ebenen angeordnet. Die Strahlenmeßanordnung (8) ist ein die Strahlenquellen (5, 6, 7) umschließender Ring aus einer Vielzahl von in Reihe zueinander liegenden Detektorelementen (z.B. 9, 10). Die Breite jedes Detektorelementes (z.B. 9, 10) ist mindestens gleich dem größtmöglichen Abstand der Fokusebenen voneinander (Fig. 1).

FIG 1

0025982

SIEMENS AKTIENGESELLSCHAFT    Unser Zeichen
Berlin und München    VPA 79 P 5085 EUR

Schichtgerät zur Herstellung von Transversalschichtbildern eines Aufnahmeobjektes

Die Erfindung betrifft ein Schichtgerät zur Herstellung von Transversalschichtbildern eines Aufnahmeobjektes mit mehreren, mit ihren Fokussen um gleich grosse Winkel gegeneinander versetzt angeordneten Strahlenquellen, mit einer Strahlenmeßanordnung, welche die
Strahlungsintensität hinter dem Objekt ermittelt, mit
einer Antriebsvorrichtung, die einen Drehrahmen für
die Strahlenquellen antreibt, sowie mit einem Meßwertumformer für die Transformation der von der Strahlenmeßanordnung gelieferten Signale in ein Schichtbild,
bei dem mindestens ein Teil der Strahlenquellen in
Richtung der Drehachse mit ihren Fokussen in im Abstand zueinander liegenden Ebenen angeordnet werden
können.

Ein Schichtgerät dieser Art ist in der deutschen Patentanmeldung 28 52 968 beschrieben. Bei diesem Schichtgerät ist für jede Röntgenröhre je ein Strahlenempfänger

Tp 5 Ler / 14.8.1979

vorhanden, der mit der Röntgenröhre zur Abtastung des
Aufnahmeobjektes gedreht wird. Es ist ein Rechner vorgesehen, der die Ausgangssignale der Strahlenempfänger,
die bei unterschiedlichen Projektionen während der
Drehung des Drehrahmens erzeugt werden, in der Weise
verarbeitet, daß daraus die Strahlenschwächungskoeffizienten bestimmter Punkte der durchstrahlten Schicht
berechnet werden, die dann bildmäßig wiedergegeben
werden können.

Bei dem bekannten Schichtgerät der eingangs genannten
Art ist die Zeit für die Untersuchung mehrerer paralleler Schichten des Patienten dadurch klein gehalten,
daß mehrere parallele Schichten des Aufnahmeobjektes
gleichzeitig abgetastet werden. Es ist also nicht erforderlich, nach der Abtastung einer Schicht die Liege
mit dem Aufnahmeobjekt um einen Schritt weiterzubewegen und eine erneute Abtastung vorzunehmen.

Aufgrund der Verwendung je eines Strahlenempfängers
für jede Strahlenquelle ergibt sich ein verhältnismäßig komplizierter und störanfälliger Aufbau, denn
die Strahlenempfänger müssen mit den Strahlenquellen
zur Einstellung der jeweiligen Lage der abgetasteten
Schichten in Richtung der Drehachse verschoben und
bei der Abtastung mit den Strahlenquellen gedreht
werden.

Der Erfindung liegt die Aufgabe zugrunde, ein Schichtgerät der eingangs genannten Art, also einen sogenannten Computer-Tomographen, gegenüber dem geschilderten
Stand der Technik in seinem Aufbau wesentlich zu vereinfachen, und zwar hinsichtlich der Detektorausbildung und -anordnung.

Diese Aufgabe ist erfindungsgemäß dadurch gelöst, daß die Strahlenmeßanordnung ein die Strahlenquellen umschließender Ring aus einer Vielzahl von in Reihe zueinander liegenden Detektorelementen ist und daß die Breite jedes Detektorelementes in Richtung der Drehachse mindestens gleich dem größtmöglichen Abstand der Fokusebenen ist. Bei dem erfindungsgemäßen Computer-Tomographen ist mittels eines einzigen, bei der Abtastung des Aufnahmeobjektes feststehenden Detektorringes die Aufnahme mehrerer zueinander paralleler Schichten des Aufnahmeobjektes aufgrund der Breite der Detektorelemente möglich.

Die Erfindung ist nachfolgend anhand eines in der Zeichnung dargestellten Ausführungsbeispiels näher erläutert. Es zeigen:

Fig. 1 eine Seitenansicht eines Schichtgerätes nach der Erfindung bei geöffnetem Gehäuse,

Fig. 2 eine Ansicht des Gerätes gemäß Figur 1 von oben bei aufgeschnittenem Drehrahmen,

Fig. 3 eine schematische Ansicht des Gerätes gemäß Figur 1 zur Erläuterung der Kabelführung für die Strahlenquellen, und

Fig. 4 eine Ansicht der Kabelführung von oben.

In den Figuren 1 und 2 ist ein Drehrahmen 1 gezeigt, der als Hohlzylinder ausgebildet ist und auf seiner äußeren Mantelfläche einen Zahnkranz 2 aufweist, in den ein Ritzel 3 eines Antriebsmotors 4 eingreift. Auf dem Drehrahmen 1 sind drei um jeweils 120$^{\circ}$ gegen-

einander versetzte Röntgenröhren 5, 6, 7 befestigt. Jede der Röntgenröhren 5 bis 7 strahlt auf einen Strahlenempfänger 8, der als die Röntgenröhren 5 bis 7 umschließender, auf der Innenseite des Drehrahmens 1 angeordneter Ring aus einer Vielzahl von in Reihe zueinander liegenden Detektorelementen ausgebildet ist. Der Strahlenempfänger 8 ist am Gehäuse 11 befestigt. In der Figur 1 sind beispielsweise zwei Detektorelemente mit 9 und 10 bezeichnet, die auch in der Figur 2 sichtbar sind. Jede der Röntgenröhren 5 bis 7 sendet ein fächerförmiges Röntgenstrahlenbündel aus, dessen Ausdehnung in Längsrichtung der Achse des Aufnahmeobjektes, das in eine Öffnung 11a des Gehäuses 11 eingebracht wird, gleich der gewählten Schichtdicke ist und dessen Breite so bemessen ist, daß die gesamte Öffnung 11a und damit das gesamte Aufnahmeobjekt erfaßt wird. Der Drehrahmen 1 ist durch den Motor 4 um eine Achse 12, mit der etwa die Längsachse des Aufnahmeobjektes zusammenfallen soll, drehbar. Die Röntgenröhren 5 bis 7 sind an einem Röntgengenerator angeschlossen, während der Strahlenempfänger 8 seine Signale einem Meßwertumformer zuführt, der daraus ein Röntgenbild aufbaut und seine Wiedergabe auf einem Sichtgerät bewirkt.

Die Röntgenröhre 5 ist am Drehrahmen 1 fest angeordnet, d.h. nicht verstellbar. Im Gegensatz dazu ist die Röntgenröhre 7 in Richtung der Drehachse 12 verstellbar am Drehrahmen 1 gelagert. Hierzu ist am Drehrahmen 1 ein Motor 13 mit einer Stellspindel 14 für die Röntgenröhre 7 befestigt. In gleicher Weise ist die Röntgenröhre 6 in Richtung der Drehachse 12 über einen am Drehrahmen 1 befestigten Motor 17 mit einer Stellspindel 18 für die Röntgenröhre 6 verstellbar.

Durch Verstellung der Röntgenröhren 6 und 7 in Richtung der Drehachse 12 kann der Abstand dieser Röntgenröhren von der Röntgenröhre 5 und damit der Abstand dreier durch eine Drehung des Drehrahmens 1 gleichzeitig abtastbarer, paralleler Körperschichten festgelegt werden. Aus der Figur 2 geht hervor, daß die Breite jedes Detektorelementes, beispielsweise der Detektorelemente 9,10 in Richtung der Drehachse 12 etwa gleich dem größtmöglichen Abstand der Ebenen, in der die Fokusse der Röntgenröhren 5, 6, 7 liegen, ist, so daß in jeder Stellung der Röntgenröhren 6, 7 deren Strahlenbündel von den Detektorelementen des Strahlenempfängers 8 erfaßt werden.

Es ist auch möglich, die Röntgenröhren 6 und 7 so zu verstellen, daß alle drei Röntgenröhren 5, 6, 7 mit ihren Fokussen in einer Ebene liegen. In diesem Fall braucht für die gleiche Bildauflösung wie bei im Abstand voneinander angeordneten Röntgenröhren nur eine Drehung von 120$^{\text{O}}$ zu erfolgen. Die Zeit für die Abtastung einer Schicht beträgt daher nur ein Drittel der Zeit, die für die Abtastung einer Schicht erforderlich ist, wenn die Röntgenröhren gemäß Figur 2 im Abstand voneinander liegen. Wird der Abtastwinkel größer als 120$^{\text{O}}$, z.B. 360$^{\text{O}}$, bei in einer Ebene liegenden Fokussen gewählt, so ergibt sich eine größere Zahl von Ausgangssignalen pro Schicht als in dem Fall, in dem die Röntgenröhren im Abstand voneinander liegen. Dadurch ist eine bessere Bildauflösung gegeben.

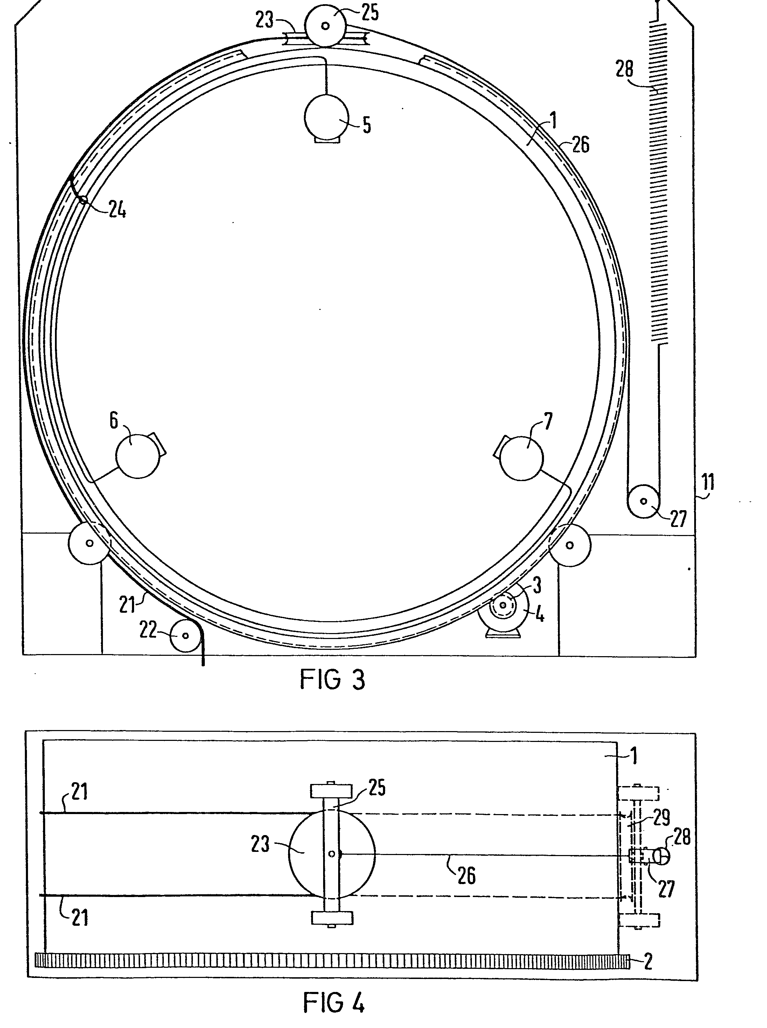

Die Zuführung der Hochspannung und gegebenenfalls der Steuersignale zu den Röntgenröhren 5 bis 7 erfolgt gemäß den Figuren 3 und 4, in denen nur die für diese Zuführung wesentlichen Teile dargestellt sind, durch vom Röntgengenerator kommende Kabel 21, die über eine im Sockel des Gerätes angeordnete Rolle 22 geführt und um den äußeren Mantel des Drehrahmens 1 gelegt sind. Von der Rolle 22 sind die Kabel dabei zu einer Rolle 23 auf dem Mantel des Drehrahmens 1 und von dort zu einem festen Punkt 24 des Drehrahmens 1 geführt. Vom Punkt 24 führt eine feste Verkabelung zu den Röntgenröhren 5, 6, 7. Die Rolle 23 ist mittels eines Wagens 25 auf dem Mantel des Drehrahmens 1 in Umfangsrichtung verschiebbar. An ihm greift ein Seilzug 26 an, der über eine gerätefeste Rolle 27 geführt und an einer Feder 28 befestigt ist, welche am Gehäuse 11 verankert ist.

Bei einer Drehung des Drehrahmens 1 läuft die Rolle 25 auf dem Mantel des Drehrahmens 1 und hält die Kabel 21 gespannt. Sie bewegt sich dabei beispielsweise in die in der Figur 4 gestrichelt gezeichnete, durch das Bezugszeichen 29 gezeichnete Stellung.

Patentanspruch

Schichtgerät zur Herstellung von Transversalschichtbildern eines Aufnahmeobjektes mit mehreren, mit ihren Fokussen um gleich große Winkel gegeneinander versetzt angeordneten Strahlenquellen, mit einer Strahlenmeßanordnung, welche die Strahlungsintensität hinter dem Objekt ermittelt, mit einer Antriebsvorrichtung, die einen Drehrahmen für die Strahlenquellen antreibt, sowie mit einem Meßwertumformer für die Transformation der von der Strahlenmeßanordnung gelieferten Signale in ein Schichtbild, bei dem mindestens ein Teil der Strahlenquellen in Richtung der Drehachse mit ihren Fokussen in im Abstand zueinander liegenden Ebenen angeordnet werden können,  d a d u r c h  g e k e n n z e i c h n e t ,  daß die Strahlenmeßannung (8) ein die Strahlenquellen (5, 6, 7) umschliessender Ring aus einer Vielzahl von in Reihe zueinander liegenden Detektorelementen (z.B. 9, 10) ist und daß die Breite jedes Detektorelementes (z.B. 9, 10) in Richtung der Drehachse (12) mindestens gleich dem größtmöglichen Abstand der Fokusebenen ist.

0025982
VPA 79 P 5085 EUR

FIG 1

FIG 2

0025982

VPA 79 P 5085 EUR

FIG 3

FIG 4